# EUROPÄISCHE PATENTSCHRIFT

(11) **EP 2 214 872 B1**
(45) Veröffentlichungstag und Bekanntmachung des Hinweises auf die Patenterteilung: **13.02.2019**
(21) Anmeldenummer: 08851939.2
(22) Anmeldetag: 18.11.2008
(51) Int. Cl.: B25J 13/00

(54) **ROBOTER UND VERFAHREN ZUM PROJIZIEREN EINES BILDES AUF DIE OBERFLÄCHE EINES OBJEKTS**
ROBOT AND METHOD FOR PROJECTING AN IMAGE ONTO THE SURFACE OF AN OBJECT
ROBOT ET PROCÉDÉ POUR PROJETER UNE IMAGE SUR LA SURFACE D'UN OBJET

(30) Priorität: 19.11.2007 DE 102007055204
(43) Veröffentlichungstag der Anmeldung: 11.08.2010
(73) Patentinhaber: KUKA Deutschland GmbH, 86165 Augsburg (DE); Deutsches Zentrum für Luft- und Raumfahrt e.V., 51147 Köln (DE)
(72) Erfinder: ORTMAIER, Tobias, 30966 Hemmingen (DE); JACOB, Dirk, 87616 Marktoberdorf (DE); PASSIG, Georg, 80639 München (DE)
(74) Vertreter: Ege Lee & Partner Patentanwälte PartGmbB
(86) Internationale Anmeldenummer: PCT/EP2008/065758
(87) Internationale Veröffentlichungsnummer: WO 2009/065831

(56) Entgegenhaltungen:
- EP-A- 1 555 507
- EP-A1- 1 555 507
- EP-A1- 1 555 507
- JP-A- 2005 165 804
- JP-A- 2008 006 551
- KR-A- 20040 056 489
- KR-A- 20040 056 489
- TERASHIMA M ET AL: "A human-robot interface using an extended digital desk", ROBOTICS AND AUTOMATION, 1999. PROCEEDINGS. 1999 IEEE INTERNATIONAL CO NFERENCE ON DETROIT, MI, USA 10-15 MAY 1999, PISCATAWAY, NJ, USA,IEEE, US, Bd. 4, 10. Mai 1999 (1999-05-10), Seiten 2874-2880, XP010336691, DOI: 10.1109/ROBOT.1999.774033 ISBN: 978-0-7803-5180-6
- TERASHIMA M ET AL: "A human-robot interface using an extended digital desk", ROBOTICS AND AUTOMATION, 1999. PROCEEDINGS. 1999 IEEE INTERNATIONAL CO NFERENCE ON DETROIT, MI, USA 10-15 MAY 1999, PISCATAWAY, NJ, USA,IEEE, US, vol. 4, 10 May 1999 (1999-05-10), pages 2874-2880, XP010336691, DOI: 10.1109/ROBOT.1999.774033 ISBN: 978-0-7803-5180-6

## Beschreibung

Die Erfindung betrifft einen Roboter und ein Verfahren zum Projizieren eines Bildes auf die Oberfläche eines Objekts.

Das Dokument TERASHIMA M ET AL: "A human-robot interface u-sing an extended digital desk", Robotics and Automation 1999, beschreibt einen digitalen Tisch, auf dessen Oberfläche mittels eines Projektors virtuelle Eingabemittel projiziert werden, mit dem Zweck einen vom separaten Tisch entfernten Roboterarm programmieren zu können, wobei zur Programmierung durch eine Person die Szene der Roboterzelle und auch der Roboter selbst auf der Oberfläche des Tisches aufprojiziert werden.

Die EP 1 555 507 A1 beschreibt einen am Endglied eines Roboterarms angebrachten Lichtschnittsensor, der ausgebildet ist Kanten und Punkte auf einem Bauteil optisch zu erfassen.

Die KR 10 2004 005 6489 A beschreibt ein Computersystem, das zur Korrektur einer Verzeichnung bei einer Projektion eines Bildes auf einer nichtebenen Oberfläche vorgesehen ist.

Beispielsweise in der Chirurgie kommen zunehmend computerbasierte Informationssysteme zum Einsatz, die einen, ein Lebewesen operierenden, Chirurgen mit Informationen versorgen.

Die Informationen können beispielsweise Vitalparameter des Lebewesens, Risikostrukturen oder Zielpositionen für die Operation sein. Die Informationen werden beispielsweise mit einer Anzeigevorrichtung des Informationssystems dargestellt und das Informationssystem kann Eingabemittel zum Bedienen desselben umfassen. Aufgrund der mittels der Anzeigevorrichtung dargestellten Informationen ist ein Blickwechsel des Chirurgen zwischen dem Operationstisch und der Anzeigevorrichtung und gegebenenfalls den Eingabemittel nötig.

Die Aufgabe der Erfindung ist es daher, eine Vorrichtung anzugeben, die Voraussetzungen für ein erleichtertes Erfassen von visuellen Informationen erlaubt.

Die Aufgabe der Erfindung wird gelöst durch einen Roboter mit einem Roboterarm und einer am Roboterarm befestigten oder in den Roboterarm integrierten Vorrichtung zum Projizieren eines Bildes auf die Oberfläche eines Objekts, eine Datenverarbeitungsvorrichtung und eine ins-besondere am Roboterarm befestigte oder in den Roboterarm integrierte Vorrichtung zum Aufnehmen eines Bildes vom auf der Oberfläche des Objekts projizierten Bild, das virtuelle Eingabemittel der Datenverarbeitungsvorrichtung oder virtuelle Eingabemittel einer weiteren Datenverarbeitungsvorrichtung darstellen, wobei die Datenverarbeitungsvorrichtung eingerichtet ist, einen Bilddatensatz zu analysieren, der einem mit der Vorrichtung zum Aufnehmen eines Bildes aufgenommenen Bild vom auf der Oberfläche des Objekts projizierten Bild zugeordnet ist und den Bilddatensatz auf eine Gestik einer Person zu analysieren, um ein Betätigen der virtuellen Eingabemittel zu erkennen.

Die Aufgabe der Erfindung wird auch gelöst durch ein Verfahren zum Projizieren eines Bildes auf die Oberfläche eines Objekts, aufweisend den folgenden Verfahrensschritt:
Projizieren eines Bildes auf die Oberfläche eines Objekts, das ein Lebewesen ist, mit einer insbesondere in einen Roboterarm eines Roboters integrierten oder an den Roboterarm befestigten Vorrichtung zum Projizieren eines Bildes,
Aufnehmen eines Bildes vom auf der Oberfläche des Lebewesens projizierten Bildes mittels einer am Roboterarm befestigten oder in den Roboterarm integrierten Kamera, wobei das projizierte Bild virtuelle Eingabemittel darstellen, und
Analysieren eines mit der Kamera aufgenommenen Bildes zugeordneten weiteren Bilddatensatzes auf eine Gestik einer Person, um ein Betätigen der virtuellen Eingabemittel zu erkennen.

Obwohl das erfindungsgemäße Verfahren bzw. der erfindungsgemäße Roboter insbesondere für das medizinische Umfeld gedacht ist, sind auch nicht medizinische Anwendungen, beispielsweise bei der Wartung, Inspektion oder Reparatur von Maschinen, denkbar.

Roboter im Allgemeinen sind Handhabungsmaschinen, die zur selbsttätigen Handhabung von Objekten mit zweckdienlichen Werkzeugen ausgerüstet und bezüglich mehrerer Bewegungsachsen insbesondere hinsichtlich Orientierung, Position und Arbeitsablauf programmierbar sind. Roboter umfassen im Allgemeinen den Roboterarm, der auch als Manipulator bezeichnet wird, eine Steuervorrichtung und gegebenenfalls einen Effektor, der z.B. als ein Greifer zum Greifen eines Werkzeugs oder, wenn in der Medizintechnik angewandt, zum Anbringen eines medizintechnischen Instrumentes ausgebildet sein kann. Der Manipulator bzw. der Roboterarm stellt im Wesentlichen den beweglichen Teil des Roboters dar. Der Roboterarm weist insbesondere mehrere Achsen auf, die z.B. mittels elektrischer Antriebe von der z.B. als Rechner ausgeführten Steuervorrichtung angesteuert werden.

Um ein Bild auf die Oberfläche des Objekts, das insbesondere ein Lebewesen ist, zu projizieren, ist erfindungsgemäß im Roboterarm des Roboters die Vorrichtung zum Projizieren des Bildes integriert bzw. an diesem befestigt. Somit ist es in relativ einfacher Weise möglich, aufgrund einer geeigneten Ansteuerung des Roboterarms das Bild auf die Oberfläche des Objekts zu projizieren. Die Vorrichtung zum Projizieren des Bildes kann insbesondere in der Nähe des sogenannten Tool-Center-Points (TCP) des Roboters integriert bzw. befestigt sein. Der Ort der Projektion kann beispielsweise durch Software gewählt werden.

Der erfindungsgemäße Roboter weist eine insbesondere am Roboterarm befestigte oder in den Roboterarm integrierte Vorrichtung zum Aufnehmen eines Bildes vom auf der Oberfläche des Objekts projizierten Bild auf, wobei eine Datenverarbeitungsvorrichtung insbesondere des Roboters eingerichtet ist, einen Bilddatensatz zu analysieren, das einem mit der Vorrichtung zum Aufnehmen eines Bildes aufgenommen Bild vom auf der Oberfläche des Objekts projizierten Bild zugeordnet ist. Die Vorrichtung zum Aufnehmen eines Bildes vom auf der Oberfläche des Objekts projizierten Bild ist z.B. eine Kamera oder Sensoren, die insbesondere von sich aus ein 2,5D Bild der Umgebung liefern. Die Vorrichtung zum Aufnehmen eines Bildes kann aber auch z.B. an einem Stativ, an einer Wand oder an einer Decke befestigt sein.

Bei dem projizierten Bild handelt es sich um virtuelle Eingabemittel der Datenverarbeitungsvorrichtung insbesondere des Roboters oder um virtuelle Eingabemittel einer weiteren Datenverarbeitungsvorrichtung, wobei die Datenverarbeitungsvorrichtung insbesondere des Roboters derart eingerichtet, dass sie den Bilddatensatz auf eine Gestik einer Person analysiert, um ein Betätigen der virtuellen Eingabemittel zu erkennen. Die virtuellen Eingabemittel können z.B. virtuelle, auf der Oberfläche des Objekts mittel des projizierten Bildes dargestellte Bedienelemente aufweisen, wobei die Datenverarbeitungsvorrichtung den Bilddatensatz beispielsweise mittels Mustererkennungsalgorithmen daraufhin analysiert, ob die Person die virtuell dargestellten Bedienelemente berührt. Aufgrund der Analyse des Bildes bzw. des Bilddatensatzes ist es demnach möglich, die Gestik der Person
zu erkennen und diese als Bedienen der virtuellen Eingabemittel zu interpretieren. Handelt es sich insbesondere bei dem Objekt um das Lebewesen, beispielsweise eines zu behandelnden Patienten, so kann die Person, die beispielsweise ein Chirurg oder allgemein ein Arzt ist, im sterilen Umfeld die virtuellen Eingabemittel betätigen.

Nach einer weiteren Ausführungsform des erfindungsgemäßen Roboters weist dieser Mittel zum Erkennen einer durch die Oberflächengeometrie der Oberfläche des Objekts bedingten Verzeichnung des projizierten Bildes auf, die die Vorrichtung zum Projizieren des Bildes derart ansteuert, damit diese die Verzeichnung des projizierten Bildes zumindest teilweise kompensiert. Unter Verzeichnung versteht man, dass die Bildhöhe, d.h. der Abstand eines Bildpunktes von der Bildmitte des projizierten Bildes auf nicht-lineare Weise von der Höhe des entsprechenden Objektpunktes abhängt. Man kann auch sagen, dass der Abbildungsmaßstab von der Höhe des entsprechenden Objektpunktes abhängt. Eine Verzeichnung bewirkt z.B., dass gerade Linien, deren Abbild nicht durch die Bildmitte des projizierten Bildes geht, gekrümmt wiedergegeben werden.

Die Mittel zum Erkennen der Verzeichnung umfassen gemäß einer Variante des erfindungsgemäßen Roboters die insbesondere in den Roboterarm integrierte oder an den Roboterarm befestigte Vorrichtung zum Aufnehmen eines Bildes und die Datenverarbeitungsvorrichtung, die gemäß dieser Ausführungsform eingerichtet ist, aufgrund des analysierten Bilddatensatzes die Verzeichnung zu erkennen. Dies kann beispielsweise folgendermaßen realisiert werden: Der dem Bild vom projizierten Bild zugeordnete Bilddatensatz kann mit dem dem projizierten Bild zugeordneten Bilddatensatz verglichen werden. Dadurch entsteht ein Stereosystem, um die Verzeichnung zu ermitteln. Die inverse Abbildung des projizierten Bildes kann dann bei der Projektion des projizierten Bildes berücksichtigt werden, sodass die Verzeichnung zumindest teilweise kompensiert werden kann.

Die Bestimmung der Verzeichnung kann beispielsweise während des Projizierens des Bildes erfolgen. Die Bestimmung der Verzeichnung kann jedoch auch z.B. während eines Kalibrierungsvorganges der Vorrichtung zum Projizieren des Bildes mittels eines Referenzbildes durchgeführt werden.

Nach einer weiteren Ausführungsform des erfindungsgemäßen Roboters ist die Datenverarbeitungsvorrichtung derart eingerichtet, dass diese aufgrund eines vorab erstellten weiteren Bilddatensatzes, insbesondere eines dreidimensionalen Bilddatensatzes vom Objekt, die Vorrichtung zum Projizieren des Bildes derart ansteuert, dass diese eine Verzeichnung des projizierten Bildes zumindest teilweise kompensiert. Handelt es sich bei dem Objekt um das Lebewesen, dann kann der weitere Bilddatensatz vom Objekt insbesondere mittels eines bildgebenden medizintechnischen Gerätes, wie beispielsweise eines Magnetresonanzgerätes, eines Computertumorgraphiegerätes, eines Röntgengerätes oder eines Ultraschallgerätes erstellt werden.

Der erfindungsgemäße Roboter ist insbesondere vorgesehen, im Rahmen eines medizinischen Arbeitsplatzes verwendet zu werden. Gemäß eines medizinischen Arbeitsplatzes weist dieser neben dem erfindungsgemäßen Roboter ein Navigationssystem zum Erfassen der Position des Lebewesens und des Roboters auf, wobei die Datenverarbeitungseinrichtung des Roboters derart eingerichtet ist, dass sie die Vorrichtung zum Projizieren des Bildes derart ansteuert, dass die Position des Roboters und des Lebewesens bei dem zumindest teilweise Kompensieren der Verzeichnung des projizierten Bildes berücksichtigt wird. Diese Variante des medizinischen Arbeitsplatzes ist demnach derart ausgeführt, dass für ein Verfahren zusätzlich folgende Verfahrensschritte ausgeführt werden können:
- Ermitteln der Position des Roboters und des Lebewesens insbesondere mittels des Navigationssystems,
- Ermitteln der zu erwartenden Verzeichnung des auf die Oberfläche des Lebewesens projizierten Bildes aufgrund der Position des Lebewesens und des Roboters und aufgrund eines vorab vom Lebewesen aufgenommen Bilddatensatzes und
- zumindest teilweise Kompensieren der erwarteten Verzeichnung des auf der Oberfläche projizierten Bildes aufgrund der Position des Roboters und des Lebewesens und aufgrund des Bilddatensatzes vom Lebewesen.

Navigationssysteme sind in der Medizintechnik, insbesondere in der minimalinversiven Medizintechnik, beispielsweise aus der DE 199 51 503 B4 bekannt. Navigationssysteme umfassen beispielsweise am Roboter angeordnete Marker und am Lebewesen oder auf einer Patientenliege, auf der das Lebewesen liegt, angeordnete Marker und eine Vorrichtung zum Erfassen der Marker. Die Vorrichtung zum Erfassen der Marker ist beispielsweise eine optische Vorrichtung, wie beispielsweise eine Stereokamera. Aufgrund der mit der Vorrichtung zum Erfassen der Marker aufgenommen Marker kann das Navigationssystem in im Wesentlichen allgemein bekannter Weise die Positionen des Roboters und des Lebewesens bestimmen. Aufgrund der Positionen des Roboters und des Lebewesens und des vom Lebewesen aufgenommenen, insbesondere dreidimensionalen Bilddatensatzes kann die Datenverarbeitungsvorrichtung die erwartete Verzeichnung des projizierten Bildes berechnen und entsprechend die Vorrichtung zum Projizieren des Bildes einstellen, sodass die erwartete Verzeichnung zumindest teilweise kompensiert wird.

Die Vorrichtung zum Erfassen der Marker des Navigationssystems kann auch dafür verwendet werden, das Bild vom projizierten Bild aufzunehmen. Der dem Bild zugeordnete Bilddatensatz kann dann auch für die Mittel zum Erkennen der Verzeichnung verwendet werden.

Wird das Navigationssystem auch während der Behandlung des Lebewesens verwendet, um dessen Position zu bestimmen, dann kann das teilweise Kompensieren der zu erwarteten Verzeichnung mit der Bewegung des Patienten aktualisiert werden. Dieses Kompensieren kann beispielsweise durch die Vorrichtung zum Projizieren als solche durchgeführt werden.

Gemäß einer Ausführungsform des erfindungsgemäßen Verfahrens wird jedoch das zumindest teilweise Kompensieren aktualisiert, indem der Roboterarm entsprechend der Bewegung des Lebewesens mitgeführt wird.

Mit dem erfindungsgemäßen Roboter bzw. mit dem erfindungsgemäßen Verfahren kann demnach eine sogenannte erweiterte Realität (Englisch: Augmented Reality) angewendet werden. Unter erweiterter Realität versteht man im Allgemeinen die computergestützte Erweiterung der Realitätswahrnehmung.

Ausführungsbeispiele der Erfindung sind exemplarisch in den beigefügten schematischen Zeichnungen dargestellt. Es zeigen:
- Fig. 1: einen Roboter,
- Fig. 2: einen den Roboter der Fig. 1 aufweisenden medizinischen Arbeitsplatz,
- Fig. 3: virtuelle Eingabemittel und
- Fig. 4: ein bildgebendes medizintechnisches Gerät.

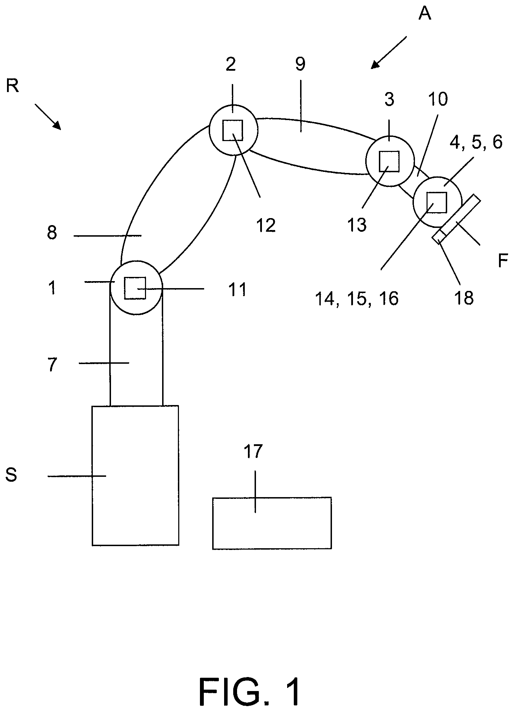

Die Fig. 1 zeigt einen Roboter R mit einem Roboterarm A, der im Falle des vorliegenden Ausführungsbeispieles auf einem Sockel S befestigt ist. Der Roboterarm A stellt im Wesentlichen den beweglichen Teil des Roboters R dar und umfasst mehrere Achsen 1 - 6, mehrere Hebel 7 - 10 und einen Flansch F, an dem z.B. ein Werkzeug oder ein medizinisches Instrument befestigt werden kann.

Jede der Achsen 1 - 6 wird im Falle des vorliegenden Ausführungsbeispieles mit einem elektrischen Antrieb bewegt, die in nicht dargestellter Weise mit einem Steuerrechner 17 des Roboters R elektrisch verbunden sind, so dass der Steuerrechner 17 bzw. ein auf dem Steuerrechner 17 laufendes Rechnerprogramm die elektrischen Antriebe derart ansteuern kann, dass die Position und Orientierung des Flansches F des Roboters R im Wesentlichen frei im Raum ausgerichtet werden kann. Die elektrischen Antriebe des Roboters R umfassen z.B. jeweils einen elektrischen Motor 11 - 16 und gegebenenfalls eine die Motoren 11 - 16 ansteuernde Leistungselektronik.

Im Falle des vorliegenden Ausführungsbeispieles ist im Roboterarm A und insbesondere im Flansch F ein Projektor 18 integriert, der auf eine Oberfläche eines Objekts ein Bild projizieren kann. Alternativ kann der Projektor 18 auch an der Struktur des Roboterarms A, insbesondere am Flansch F beispielsweise auch lösbar befestigt sein.

Der Projektor 18 ist mit einem Rechner, beispielsweise mit dem Steuerrechner 17 in nicht dargestellter Weise elektrisch verbunden.

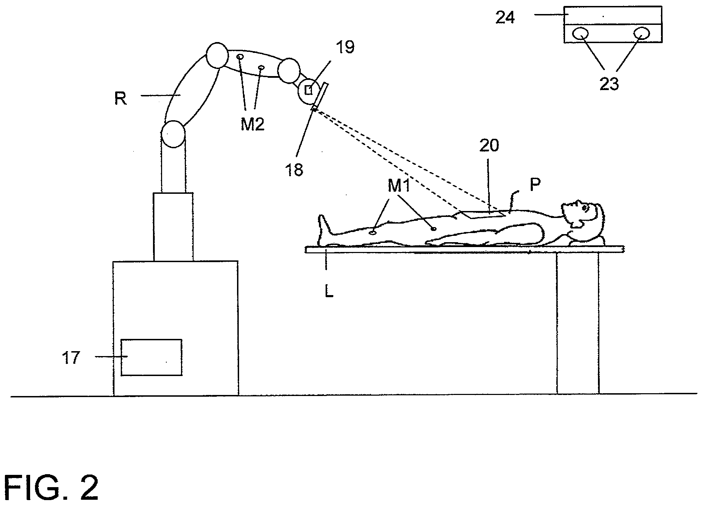

Im Falle des vorliegenden Ausführungsbeispiels ist der Roboter R vorgesehen, Teil eines in der Fig. 2 dargestellten medizinischen Arbeitsplatzes zu sein.

Der im Falle des vorliegenden Ausführungsbeispiels dargestellte medizinische Arbeitsplatz umfasst neben dem Roboter R eine Patientenliege L, die gegebenenfalls auch höhenverstellbar ist, und ein Navigationssystem. Die Patientenliege L ist vorgesehen, dass auf dieser ein in der Fig. 2 dargestellter Patient P liegen kann. Dieser kann beispielsweise von einem in der Figur 2 nicht dargestellten Chirurgen operiert werden.

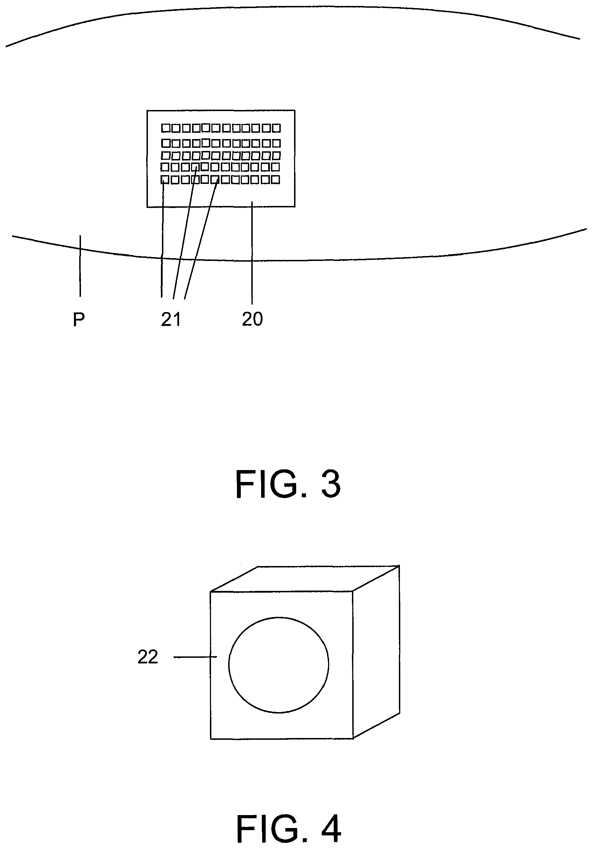

Der Roboter R ist im Falle des vorliegenden Ausführungsbeispiels vorgesehen, ein Bild 20 mittels seines Projektors 18 auf die Oberfläche des Patienten P zu projizieren. Das projizierte Bild 20, das in der Fig. 3 näher dargestellt ist, stellt im Falle des vorliegenden Ausführungsbeispiels Eingabemittel für den Steuerrechner 17 oder für eine weitere, in den Figuren nicht näher dargestellte Datenverarbeitungseinrichtung dar, und hat zum Beispiel die Form einer Tastatur. Die virtuellen Eingabemittel in Form des projizierten Bildes 20 umfassen ferner virtuelle Bedienelemente 21, die beispielsweise den Tasten einer realen Tastatur entsprechen.

Im Falle des vorliegenden Ausführungsbeispieles umfasst der Roboter R zusätzlich eine mit dem Steuerrechner 17 elektrisch verbundene Kamera 19, die im Roboterarm A integriert oder an diesem befestigt ist. Die Kamera 19 ist derart ausgerichtet, dass sie ein Bild von dem auf den Patienten P projizierten Bild 20 aufnimmt. Der dem Bild vom projizierten Bild 20 zugeordnete Bilddatensatz wird vom Steuerrechner 17 bzw. von einem auf dem Steuerrechner 17 laufenden Rechnerprogramm mittels Mustererkennungsalgorithmen dahingehend analysiert, ob eine in den Figuren nicht dargestellte Person, beispielsweise der den Patienten P operierende Chirurg, die virtuellen Bedienelemente 21 der virtuellen, in Form des projizierten Bilds 20 dargestellten virtuellen Eingabemittel betätigt. Erkennt das auf dem Steuerrechner 17 laufende Rechnerprogramm aufgrund der Analyse des eben erwähnten Bilddatensatzes, dass der Chirurg beispielsweise den Bereich eines der virtuellen Bedienelemente 21 berührt, so veranlasst das auf dem Steuerrechner 17 laufende Rechnerprogramm einer der Betätigung dieses virtuellen Bedienelementes 21 zugeordneten Reaktion.

Durch die gekrümmte Oberfläche des Patienten P weist das projizierte Bild 20 eine Verzeichnung auf. Diese wird im Falle des vorliegenden Ausführungsbeispiels zumindest teilweise dadurch kompensiert, indem vor dem Projizieren des Bildes 20 auf den Patienten P ein in den Figuren nicht näher dargestelltes Referenzbild mit dem Projektor 18 auf den Patienten P projiziert wird. Dabei wird das Referenzbild im Wesentlichen auf dieselbe Stelle auf der Oberfläche des Patienten P projiziert, auf der auch das Bild 20 projiziert wird.

Mit der Kamera 19 wird anschließend ein Bild vom projizierten Referenzbild aufgenommen und der diesem Bild zugehörige Bilddatensatz mit einem weiteren, auf dem Steuerrechner 17 laufenden Rechnerprogramm analysiert. Aufgrund des dem Bild vom projizierten Referenzbild zugeordneten Bilddatensatzes und einer Solldarstellung des projizierten Referenzbildes kann der Steuerrechner 17 den Projektor 18 derart ansteuern, dass dieser eine Verzeichnung des später abzubildenden projizierten Bildes 20 zumindest teilweise kompensiert.

In einer weiteren Ausführungsform wurde vor der Operation des Patienten P von diesem ein insbesondere dreidimensionaler Bilddatensatz mittels eines in der Fig. 4 dargestellten bildgebenden medizintechnischen Geräts 22 erstellt. Das medizintechnische Gerät 22 ist beispielsweise ein Magnetresonanzgerät, ein Computertomographiegerät, ein Röntgengerät, beispielsweise ein C-Bogen-Röntgegengerät, oder ein Ultraschallgerät.

Der von dem Patienten P erstellte dreidimensionale Bilddatensatz kann beispielsweise zur Diagnostik des Patienten P verwendet werden und wird in den Steuerrechner 17 des Roboters R eingespielt. Aufgrund der Positionen des Roboters R und des Patienten P, sowie aufgrund des dreidimensionalen Bilddatensatzes des Patienten P kann ein weiteres, auf dem Steuerrechner 17 laufendes Rechnerprogramm eine zu erwartende Verzeichnung des projizierten Bildes 20 bestimmen und dadurch die Projektion des Projektors 18 derart verändern, dass die zu erwartende Verzeichnung des projizierten Bildes 20 zumindest teilweise kompensiert wird.

Um die Positionen des Roboters R und des Patienten P zu ermitteln, wird das oben genannte Navigationssystem verwendet. Navigationssysteme sind dem Fachmann im Allgemeinen bekannt und umfassen beispielsweise eine Stereokamera 23, an dem Patienten P angeordnete Referenzmarken M1 und am Roboter R angeordnete Referenzmarken M2. Für die Bestimmung der Positionen des Roboters R und des Patienten P, nimmt die Stereokamera 28 Bilder der Referenzmarken M1, M2 auf, deren zugeordnete Bilddatensätze mittels eines Rechners 24 des Navigationssystems in allgemein bekannter Weise ausgewertet werden. Der Rechner 24 des Navigationssystems ist ferner in nicht dargestellter Weise mit dem Steuerrechner 17 des Roboters R verbunden, so dass der Steuerrechner 17 eine Information über die Positionen des Roboters R, insbesondere dessen Projektors 18, und der Position des Patienten P zur Verfügung steht.

Im Falle des vorliegenden Ausführungsbeispiels ist es außerdem vorgesehen, dass eine sich ändernde Verzeichnung des projizierten Bildes 20 aufgrund einer Bewegung des Patienten P korrigiert wird, indem beispielsweise der Projektor 18 aufgrund der erkannten Bewegung bzw. die durch die Bewegung bedingte geänderte Position des Patienten P nachstellt oder den Roboterarm A derart mit der Bewegung des Patienten P mitführt, so dass die sich ändernde Verzeichnung zumindest teilweise kompensiert wird.

## Patentansprüche

1. Roboter, aufweisend einen Roboterarm (A), eine am Roboterarm (A) befestigte oder in den Roboterarm (A) integrierte Vorrichtung (18) zum Projizieren eines Bildes (20) auf die Oberfläche eines Objekts (P), eine Datenverarbeitungsvorrichtung (17) und eine insbesondere am Roboterarm (A) befestigte oder in den Roboterarm (A) integrierte Vorrichtung zum Aufnehmen eines Bildes (19) vom auf der Oberfläche des Objekts (P) projizierten Bild (20), das virtuelle Eingabemittel der Datenverarbeitungsvorrichtung (17) oder virtuelle Eingabemittel einer weiteren Datenverarbeitungsvorrichtung darstellen, wobei die Datenverarbeitungsvorrichtung (17) eingerichtet ist, einen Bilddatensatz zu analysieren, der einem mit der Vorrichtung zum Aufnehmen eines Bildes (19) aufgenommenen Bild vom auf der Oberfläche des Objekts (P) projizierten Bild (20) zugeordnet ist und den Bilddatensatz auf eine Gestik einer Person zu analysieren, um ein Betätigen der virtuellen Eingabemittel zu erkennen.

2. Roboter nach Anspruch 1, aufweisend Mittel zum Erkennen (19, 17) einer durch die Oberflächengeometrie der Oberfläche des Objekts (P) bedingten Verzeichnung des projizierten Bildes (20), die die Vorrichtung (18) zum Projizieren des Bildes derart anzusteuern, damit diese die Verzeichnung des projizierten Bildes (20) zumindest teilweise kompensiert.

3. Roboter nach Anspruch 2, bei dem die Mittel zum Erkennen der Verzeichnung die insbesondere in den Roboterarm (A) integrierte oder an den Roboterarm (A) befestigte Vorrichtung zum Aufnehmen eines Bildes (19) und die Datenverarbeitungsvorrichtung (17) umfassen, wobei die Datenverarbeitungsvorrichtung (17) eingerichtet ist, aufgrund des analysierten Bilddatensatzes die Verzeichnung zu erkennen.

4. Roboter nach einem der Ansprüche 1 bis 3, dessen Datenverarbeitungsvorrichtung (17) eingerichtet ist, aufgrund eines vorab erstellten weiteren Bilddatensatzes, insbesondere eines dreidimensionalen Bilddatensatzes vom Objekt (P) die Vorrichtung zum Projizieren des Bildes derart anzusteuern, dass diese eine Verzeichnung des projizierten Bildes (20) zumindest teilweise kompensiert.

5. Roboter nach Anspruch 4, bei dem das Objekt ein Lebewesen (P) ist und der weitere Bilddatensatz vom Objekt mittels eines bildgebenden medizintechnischen Gerätes (22) erstellt wurde.

6. Verfahren zum Projizieren eines Bildes auf die Oberfläche eines Objekts, aufweisend folgenden Verfahrensschritt:
- Projizieren eines Bildes (20) auf die Oberfläche eines Objekts, das ein Lebewesen (P) ist, mit einer insbesondere in einen Roboterarm (A) eines Roboters (R) integrierten oder an den Roboterarm (A) befestigten Vorrichtung (18) zum Projizieren eines Bildes,
- Aufnehmen eines Bildes vom auf der Oberfläche des Lebewesens (P) projizierten Bildes (20) mittels einer am Roboterarm (A) befestigten oder in den Roboterarm (A) integrierten Kamera (19), wobei das projizierte Bild (20) virtuelle Eingabemittel darstellen, und
- Analysieren eines mit der Kamera (19) aufgenommenen Bildes zugeordneten weiteren Bilddatensatzes auf eine Gestik einer Person, um ein Betätigen der virtuellen Eingabemittel zu erkennen.

7. Verfahren nach Anspruch 6, zusätzlich aufweisend folgende Verfahrensschritte:
- Ermitteln der Positionen des Roboters (R) und des Lebewesens (P) insbesondere mittels eines Navigationssystems (23, 24. M1, M2),
- Ermitteln einer zu erwartenden Verzeichnung des auf die Oberfläche des Lebewesens (P) projizierten Bildes (20) aufgrund der Positionen des Lebewesens (P) und des Roboters (R) und aufgrund eines vorab vom Lebewesen (P) aufgenommenen Bilddatensatzes und
- zumindest teilweise Kompensieren der erwarteten Verzeichnung des auf die Oberfläche projizierten Bildes (20) aufgrund der Positionen des Roboters (R) und des Lebewesens (P) und aufgrund des Bilddatensatzes vom Lebewesen (P).

8. Verfahren nach Anspruch 7, aufweisend Aktualisieren des zumindest teilweisen Kompensierens der zu erwartenden Verzeichnung des auf die Oberfläche projizierten Bildes (20) aufgrund einer Bewegung des Lebewesens (P).

9. Verfahren nach Anspruch 8, aufweisend Aktualisieren des zumindest teilweisen Kompensierens der zu erwartenden Verzeichnung des auf die Oberfläche projizierten Bildes (20) aufgrund der Bewegung des Lebewesens (P) durch ein der Bewegung entsprechendes Mitführen des Roboterarms (A) .

## Claims

1. Robot comprising a robot arm (A), a device (18) secured to the robot arm (A) or integrated into the robot arm (A) for projecting an image (20) onto the surface of an object (P), a data processing device (17) and a device secured in particular to the robot arm (A) or integrated into the robot arm (A) for capturing an image (19) of the image (20) projected onto the surface of the object (P), which virtual input means of the data processing device (17) or virtual input means of an additional data processing device present, wherein the data processing device (17) is configured to analyse an image data record which is assigned to an image captured by the device for capturing an image (19) of the image (20) projected onto the surface of the object (P) and to analyse the image data record for a gesture of a person in order to identify the activation of the virtual input means.

2. Robot according to claim 1, comprising means (19, 17) for detecting a distortion of the projected image (20) determined by the surface geometry of the surface of the object (P), which means control the device (18) for projecting the image such that the latter compensates at least partly for the distortion of the projected image (20).

3. Robot according to claim 2, wherein the means for detecting the distortion comprise the device integrated in particular into the robot arm (A) or secured onto the robot arm (A) for capturing an image (19) and the data processing device (17), wherein the data processing device (17) is configured to detect the distortion on the basis of the analysed image data record.

4. Robot according to any of claims 1 to 3, the data processing device (17) of which is configured on the basis of a previously generated additional image data record, in particular a three-dimensional image data record of the object (P) to actuate the device for projecting the image such that the latter at least partially compensates for a distortion of the image (20).

5. Robot according to claim 4, wherein the object is a living being (P) and the additional image data record of the object was generated by a medical imaging device (22).

6. Method for projecting an image onto the surface of an object, comprising the following method step:
- projecting an image (20) onto the surface of an object, which is a living being (P), with a device (18) for projecting an image integrated in particular into a robot arm (A) of a robot (R) or secured onto the robot arm (A),
- capturing an image of an image (20) projected onto the surface of the living being (P) by means of a camera (19) secured onto the robot arm (A) or integrated into the robot arm (A), wherein the projected images (20) represent virtual input means and
- analysing an image captured by the camera (19) of an assigned additional image data record for a gesture of a person in order to recognise the activation of the virtual input means.

7. Method according to claim 6, additionally comprising the following method steps:
- determining the positions of the robot (R) and the living being (P) in particular by means of a navigation system (23, 24, M1, M2),
- determining an anticipated distortion of the image (20) projected onto the surface of the living being (P) based on the positions of the living being (P) and the robot (R) and on the basis of a previously captured image data record of the living being (P) and
- at least partly compensating the anticipated distortion of the image (20) projected onto the surface on the basis of the positions of the robot (R) and the living being (P) and on the basis of the image data record of the living being (P).

8. Method according to claim 7, comprising updating the at least partial compensation of the anticipated distortion of the image (20) projected onto the surface caused by a movement of the living being (P).

9. Method according to claim 8, comprising updating the at least partial compensation of the distortion to be anticipated of the image (20) projected onto the surface on the basis of the movement of the living being (P) by a movement of the robot arm corresponding to the movement.

## Revendications

1. Robot, présentant un bras de robot (A), un dispositif (18) fixé sur le bras de robot (A) ou intégré dans le bras de robot (A) pour projeter une image (20) sur la surface d'un objet (P), un dispositif de traitement de données (17) et un dispositif, en particulier fixé sur le bras de robot (A) ou intégré dans le bras de robot (A), pour la capture d'une image (19) de l'image (20) projetée sur la surface de l'objet (P) que représentent des moyens d'entrée virtuels dudit dispositif de traitement de données (17) ou des moyens d'entrée virtuels d'un autre dispositif de traitement de données, le dispositif de traitement de données (17) étant prévu pour analyser un jeu de données d'images qui est associé à une image capturée avec le dispositif de capture d'une image (19) de l'image (20) projetée sur la surface de l'objet (P) et pour analyser le jeu de données d'image en ce qui concerne les gestes d'une personne, pour reconnaître un actionnement des moyens d'entrée virtuels.

2. Robot selon la revendication 1, présentant des moyens de reconnaissance (19, 17) d'une distorsion de l'image (20) projetée due à la géométrie de surface de la surface de l'objet (P), qui pilotent le dispositif (18) de projection de l'image de telle sorte pour que celle-ci compense au moins partiellement la distorsion de l'image projetée (20).

3. Robot selon la revendication 2, dans lequel les moyens de reconnaissance de la distorsion comprennent le dispositif de capture d'une image (19), en particulier intégré dans le bras de robot (A) ou fixé sur le bras de robot (A), et le dispositif de traitement de données (17), le dispositif de traitement de données (17) étant prévu pour reconnaître la distorsion sur la base du jeu de données d'images analysé.

4. Robot selon l'une des revendications 1 à 3, dont le dispositif de traitement de données (17) est prévu pour piloter le dispositif de projection de l'image, sur la base d'un autre jeu de données d'images créé auparavant, en particulier d'un jeu de données d'images tridimensionnel de l'objet (P), de telle sorte que celui-ci compense au moins partiellement une distorsion de l'image (20) projetée.

5. Robot selon la revendication 4, dans lequel l'objet est un être vivant (P) et l'autre jeu de données d'images de l'objet a été créé au moyen d'un appareil médical d'imagerie (22).

6. Procédé de projection d'une image sur la surface d'un objet, présentant les étapes de procédé suivantes :
- projection d'une image (20) sur la surface d'un objet qui est un être vivant (P), avec un dispositif (18) de projection d'une image qui est en particulier intégré dans un bras de robot (A) d'un robot ou fixé sur le bras de robot (A),
- capture d'une image de l'image (20) projetée sur la surface de l'être vivant (P) au moyen d'une caméra (19) fixée sur le bras de robot (A) ou intégrée dans le bras de robot (A), des moyens d'entrée représentant l'image projetée (20), et
- analyse d'un autre jeu de données d'image associé à une image capturée avec la caméra (19) en ce qui concerne les gestes d'une personne, pour reconnaître un actionnement des moyens d'entrée virtuels.

7. Procédé selon la revendication 6, présentant en outre les étapes de procédé suivantes :
- détermination des positions du robot (R) et de l'être vivant (P) en particulier au moyen d'un système de navigation (23, 24, M1, M2),
- détermination d'une distorsion à attendre de l'image (20) projetée sur la surface de l'être vivant (P) sur la base des positions de l'être vivant (P) et du robot (R) et sur la base d'un jeu de données d'images de l'être vivant (P) enregistré auparavant, et
- compensation au moins partielle de la distorsion à attendre de l'image (20) projetée sur la surface, sur la base des positions du robot (R) et de l'être vivant (P) et sur la base du jeu de données d'images de l'être vivant (P).

8. Procédé selon la revendication 7, présentant l'actualisation de ladite compensation au moins partielle de la distorsion à attendre de l'image (20) projetée sur la surface, sur la base d'un mouvement de l'être vivant (P).

9. Procédé selon la revendication 8, présentant l'actualisation de ladite compensation au moins partielle de la distorsion à attendre de l'image (20) projetée sur la surface, sur la base du mouvement de l'être vivant (P), par un guidage du bras de robot (A) correspondant au mouvement.
